# EUROPEAN PATENT APPLICATION

(11) **EP 2 100 632 A1**
(43) Date of publication of application: **16.09.2009**
(21) Application number: 08152580.0
(22) Date of filing: 11.03.2008
(51) Int. Cl.: A61M 3/02, A61M 31/00

(54) **Applicator device for body cavity**

(71) Applicant: Pantarhei Devices B.V., 3701 CH Zeist (NL)
(72) Inventor: Coelingh Bennink, Herman Jan Tijmen, 3985 MK, Werkhoven (NL); Wiegerinck, Martinus Antonius Hermanus Maria, 5613 CJ, Eindhoven (NL)
(74) Representative: Ketelaars, Maarten F.J.M.

(57) **Abstract**

Applicator device for delivery of a preparation substance to a body cavity. A tubular body (1) is provided with openings (8) in a distal end (15) of the tubular body (1), and with a connecting opening (12) for connecting a container assembly (14) in communication with the tubular body (1). The connecting opening (12) is positioned at a proximal end (16) of the tubular body (1) remote from the distal end (15), and the tubular body (1) is provided with a lumen (7) connecting the openings (8) to the connecting opening (12). A diameter (d2) of the lumen (7) is equal to or smaller than one third of a diameter (d1) of the tubular body (1) at the proximal end (16).

## Description

### Field of the invention

The present invention relates to an applicator device for delivery of a preparation substance to a body cavity, comprising a tubular body provided with openings in a distal end of the tubular body, and a connecting opening for connecting a container assembly in communication with the tubular body.

### Prior art

European patent application EP-A-0 761 246 discloses an apparatus for applying a medical fluid into body cavities. It comprises a pump device, a distribution body and a connecting tube between the pump device and the distribution body. The diameter of distribution body and connecting tube is different.

International patent application WO2006/016869 discloses a delivery system comprising a tubular body with a plunger to expel an emulsion through slits in a distal end of the tubular body. The emulsion is stored inside the tubular body next to the slits.

### Summary of the invention

The present invention seeks to provide an applicator which is specifically suited for delivery of treatment substances in a body cavity under controlled circumstances.

According to the present invention, an applicator device according to the preamble defined above is provided, in which the connecting opening is positioned at a proximal end of the tubular body remote from the distal end, and the tubular body is provided with a lumen connecting the openings to the connecting opening, a diameter of the lumen being equal to or smaller than one third of a diameter of the tubular body at the proximal end. Using such a ratio of diameter of the lumen and the tubular body allows having a minimum volume of treatment substance inside the applicator device in use, while the applicator device is very suited for delivery of treatment substances deep in a body cavity due to the length of the tubular body. This applicator device also allows keeping the container assembly which holds the treatment substance outside of the body cavity involved. The diameter of the lumen is e.g. less than or equal to 3mm, e.g. less than or equal to 2.2mm. This provides a low volume of treatment substance, and also allows a sufficient low force for delivery of the treatment substance.

In a further embodiment, the applicator device further comprises a plurality of channels at an angle with a longitudinal axis of the tubular body, which channels flow from the lumen out in the openings. This allows having a distributed delivery of the treatment substance at the distal end of the tubular body. In a further embodiment, a plurality of groups of channels is provided, in which each group of channels is at a different angle with a longitudinal axis of the tubular body. This provides for an even better distribution of delivery of the treatment substance in the body cavity. A diameter of the channels is, in an embodiment, less than or equal to 1mm. In combination with the total number of channels this provides a sufficiently low resistance, e.g. comparable to the resistance provided by the lumen.

The container assembly comprises a bellows type of container in a further embodiment. This allows efficient and easy actuation of the container by the user.

In a further embodiment, the tubular body and container assembly further comprise a disconnectable attachment assembly, e.g. a screw mount assembly. This allows for easy mating of the container assembly to the tubular body of the applicator device, at a production facility, or at a location of use of the applicator device. This also allows for possible re-use of the tubular body. In an alternative embodiment, the tubular body and container assembly are fixedly attached to each other. At production this allows to minimize the cost of elements and production steps.

The connecting opening in the proximal end of the tubular body may be used to directly insert a container assembly, e.g. in the form of a syringe. Alternatively, the connecting opening provided with a Luer lock, which in itself is known to the skilled person. Using a Luer lock, the applicator device may be used more than once, e.g. for treatment with multiple treatment substances..

In a further embodiment, the tubular body further comprises a penetrating needle in the connecting opening. This can be used for penetrating a seal in the container assembly, which allows keeping the treatment substance in the container assembly safe.

The lumen of the applicator device further comprises a valve in a further embodiment. The valve may be a one way valve, which allows for delivery of substance only, and safeguards against reflow of the treatment substance when e.g. pressure on the bellows container is released. In an alternative embodiment, the valve may be a two way valve, which allows preventing leakage of treatment substance from the container into the lumen of the tubular body during storage and prior to application and allows to suck back a sample of expulsed treatment substance into container.

In a further aspect, the present invention relates to the use of an applicator device according to the present invention embodiments for taking a sample from a body cavity. E.g. the container assembly may be filled with a fluid, which is expelled into a body cavity. This loosens cells or tissue fragments in the body cavity. The combination of fluid and cells or tissue fragments may then again be sucked up by releasing the pressure on the container assembly.

### Short description of drawings

The present invention will be discussed in more detail below, using a number of exemplary embodiments, with reference to the attached drawings, in which
Fig. 1 shows a cross sectional view of a first embodiment of the applicator device according to the present invention;
Fig. 2a and 2b show cross sectional views of details of the distal end of the tubular body of the applicator device of Fig. 1;
Fig. 3 shows a cross sectional view of a second embodiment of the applicator device according to the present invention;
Fig. 4 shows a cross sectional view of a further embodiment of the applicator device; and
Fig. 5 shows a cross sectional view of an even further embodiment of the applicator device.

### Detailed description of exemplary embodiments

The present invention relates to an applicator device or applicator 10 which can be used for therapeutic treatments, e.g. for treatment of infections in the cervix or uterus using a treatment substance, e.g. a gel or aqueous solution with an active ingredient. The treatment substance may be a pharmaceutical preparation (e.g. antibiotics, antimycotics, microbicides, monoclonal antibodies, hormones, prostaglandins, pH-regulators, bioadhesive drugs), a topical formulation, a washing fluid, a disinfection solution, an oil-based substance, a gel, a cream, a lubricant etc. More general, the applicator 10 can be used in any body cavity, e.g. a mucosal cavity, such as a vaginal cavity, rectum, throat etc.

The applicator 10 is intended for single use, although the use may comprise a periodic treatment over a limited time period, e.g. a week. For most treatments it is sufficient to dispense a limited amount of treatment substance (e.g. 3-5 ml) each time. The applicator 10 is intended and suitable for self-use, i.e. no medical staff is needed for the treatment using the applicator 10.

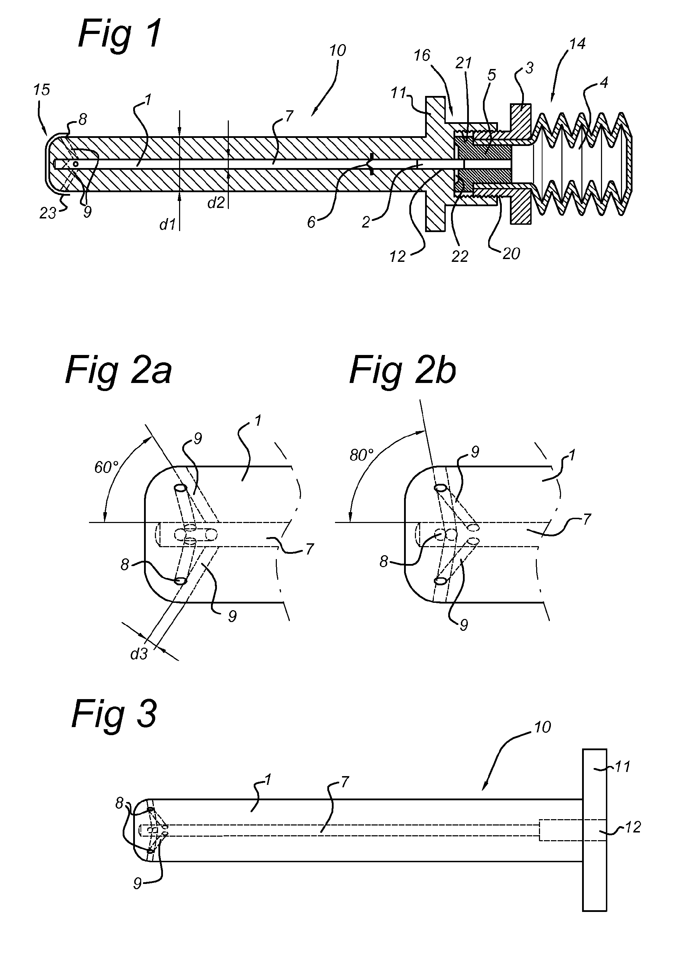

A first embodiment of the applicator 10 according to the present invention is shown in a cross sectional view in Fig. 1. The applicator comprises a tubular body 1 of a rigid material, having a centrally positioned lumen 7. The tubular body 1 further is provided with openings 8 at a distal end 15 of the tubular body 1, which openings 8 are in communication with the lumen 7 via a plurality of channels 9. At the other end (proximal end 31) of the tubular body 1, a rim 11 is provided, and a penetrating needle 2 is attached to or in a connecting opening 12 of the lumen 7. Furthermore, at this proximal end 16, the tubular body 1 comprises an attachment part 20 as part of a disconnectable attachment assembly for attaching a container assembly 14 to the tubular body 1.

The container assembly 14 comprises a container 4 in which a treatment substance, such as a preparation to be applied (fluid based, gel based...) can be stored. The container 4 is attached to a combination of handling part 3 and a connector part 5 which firmly attaches the container 4 to the handling part 3. The handling part 3 is e.g. provided with a screwing rim, which allows for easy handling and attachment to the tubular body 1. The container assembly 14 (comprising container 4, screw part 3 and connector part 5) can be attached to the tubular body 1, e.g. as shown using a screw thread connection 21 in the attachment part 20 and handling part 3, which form the disconnectable attachment assembly. Other types of connecting or attachment mechanisms may also be used in further embodiments, e.g. using a bayonet fitting or the like.

The connector part 5 may be provided with a sealing 22 which is opened by the penetrating needle 2 only when the container assembly 14 is attached to the tubular body 1.

As shown in the embodiment of Fig. 1, the container 4 is provided as a bellows type container, made of flexible material, which in combination with the rim 11 allows for an easy expulsion of the treatment substance in the container 4 into the lumen 7 in use. Further alternatives may be provided, such as a balloon type of container 4, a flexible tube type of container 4, etc.

The lumen 7 may be provided with a one way valve 6, e.g. in the form of flexible flaps. This allows preventing the treatment substance from being sucked back into the container 4 once delivered in the body cavity.

In a further alternative the lumen 7 may be provided with a two way valve 6, e.g. in the form of flexible flaps. This allows preventing leaking of fluid substance from the container into the lumen of the tubular body 1 during storage and prior to application and allows sucking back a sample of expulsed fluid substance into container 4.

The tubular body 1 has a diameter d1 (see Fig. 1) of at least 0.8 cm, e.g. equal to or larger than 1 cm. In this embodiment, the tubular body 1 is cylindrical, i.e. the diameter d1 is constant over the entire length of the tubular body 1. This allows a comfortable use as an applicator in body cavities, such as a human vaginal cavity. This thickness allows for a good sealing function of the tubular body within the body cavity. In further embodiments, the thickness d1 is even larger to improver this sealing function, e.g. more than 1.5 cm, or even more than 2.5 cm. The introduction end of the tubular body 1 is rounded off, which also improves a comfortable use of the applicator 10. In combination, the rounded off and thick nature of the tubular body 1 ensures that risk of damaging tissue in the body cavity is limited as much as possible.

The container assembly 14 is positioned remote from the delivery openings 8, which allows maintaining the treatment substance to be applied in a better controlled environment, i.e. outside of the body cavity. The container 4 is also positioned at a certain distance from the openings 8 in the tubular body 1 by having the length of the tubular body 1 to be more than a predetermined distance, i.e. more than 10cm or even more than 15 cm. This allows bringing in the applicator 10 sufficiently far in a vaginal cavity for application of a treatment substance near the uterus or cervix, while still allowing a container 4 with treatment substance to remain outside of the body cavity concerned. This also eases the operation of emptying the container 4.

In order to be able to provide as much as possible volume of the treatment substance through the openings 8 into the body cavity, a diameter d2 of the lumen 7 is small compared to an outer diameter d1 of the tubular body 1 (see Fig. 1), e.g. no more than one third of the diameter d1 of the tubular body 1. However, it must also be assured that the force needed to expulse the treatment substance from the container 4 is not too large, especially when gel type substances are to be used. In one embodiment, the diameter d2 of the lumen 7 is smaller than 5 mm, e.g. less than or equal to 3 mm or even less than or equal to 2.2mm. In one example, the diameter d1 of the tubular body at the proximal end 16 is 1 cm, and the diameter d2 of the lumen 7 is 3 mm, resulting in a ratio D2/d1 equal to 0.3 (which is less than 1/3). By having the ratio of diameters d2/d1 in the range of 1/3 or less, a good result is obtained both to have as little as possible volume of treatment substance inside the lumen 7 and channels 9, and to have a sufficiently low force needed to expel the treatment substance from the applicator 10.

The channels 9 between the lumen 7 and the openings 8 are shown in more detail in the views of Fig. 2a and 2b. In the embodiment shown, the tubular body 1 is provided with three channels 9 which are at an angle of 60° to a longitudinal axis of the tubular body 1 (or lumen 7), and three further channels 9 which are at an angle of 80° with that longitudinal axis. The openings 8 are provided at the, curved, distal end (or introduction end) of the tubular body 1 to allow for a well distributed application of the preparation in the body cavity. Using the various angles of the channels 9, the preparation is well and evenly distributed at the intended position inside the body cavity. It will be clear that further embodiments may comprise a plurality of channels 9 at a same angle with the longitudinal axis of the lumen 7, or several groups of channels 9 at different angle with the longitudinal axis of the lumen 7.

In the embodiment shown, a diameter d3 (see Fig. 2a) of each channel 9 is about 1 mm, which in combination with the number of channels 9 allows for a relatively force free expulsion of the treatment substance, while minimizing the total volume of the preparation left in the applicator 10.

The embodiment shown in Fig. 1 and 2 allows to the re-use the tubular body 1, by removing the container assembly 14 and attaching a new container assembly 14, e.g. for the daily administering of a treatment substance. In a further embodiment, the applicator 10 is used only once (although it may be applied a number of times when the container 4 is not emptied at once). In this case, the container 4 may be fixedly attached to the tubular body 1, e.g. using gluing or other permanent attachment techniques. In this case, the penetrating needle 2, and screw thread attachment 21 may be omitted altogether. In this embodiment, the tubular body 1 may be provided with a sealing cap 23 at the distal end, as shown in Fig. 1, which can be used to close off the applicator 10 before and after use.

A further embodiment of the applicator according to the present invention is shown in cross section in Fig. 3. In this case, the tubular body 1 is provided only with a rim 11, and a connecting opening 12 in communication with the lumen 7. The lumen 7 is again centrally located in the tubular body 1, and channels 9 from the lumen 7 to openings 8 are provided in a similar manner as in the embodiment described above. A number of different types of containers 4 or container assemblies may be used in combination with the applicator embodiment as shown in Fig. 3. It is e.g. possible to use a container with an exit opening adapted to the connecting opening 12, or to use a barrel and plunger type of container assembly 4 (e.g. a syringe).

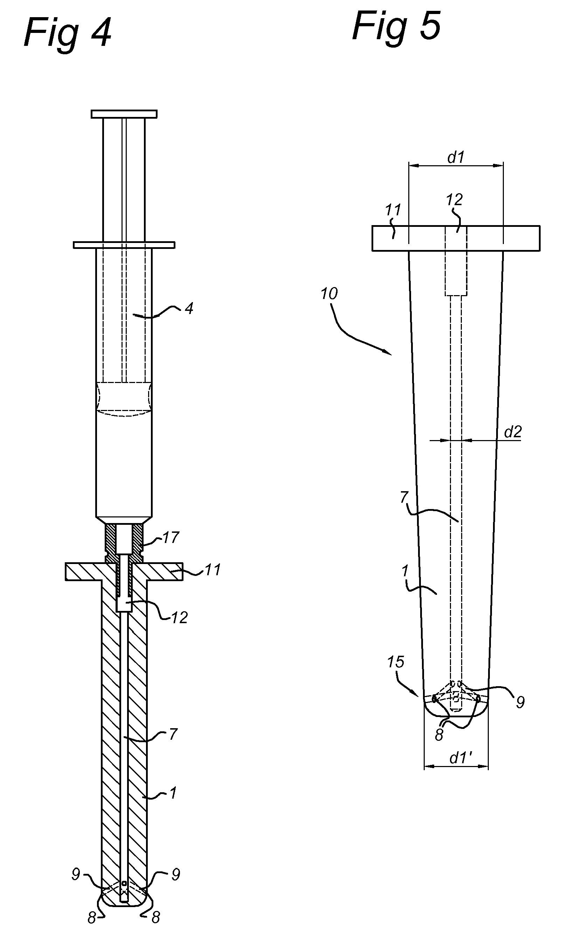

In the cross sectional view of Fig. 4, a complete applicator 10 is shown using the embodiment of the tubular body 1 of Fig. 3. The connecting opening 12 is in this embodiment furthermore provided with a Luer lock 17, which allows connecting and disconnecting a syringe type of container 4, as shown in Fig. 4. This allows to re-use the tubular body 1 multiple times with different syringe containers 4, or to use multiple syringe type containers 4 serially, e.g. to deliver different types of treatment substances into the body cavity sequentially.

A secondary use of the applicator 10 is the use as a sampler device, again for various body cavities. E.g. the applicator 10 may comprise a volume of washing fluid (e.g. a saline solution) in the container 4, and after dispensing the washing fluid in a body cavity (vaginal cavity, rectum,...) and waiting for a period of time, the washing fluid is sampled by suction into the applicator 10, but this time with cellular or tissue samples originating from that body cavity. For this use, it is important that the tubular body 1 of the applicator 10 is sufficiently thick to ensure effective sealing of the body cavity, i.e. at least 1 cm in diameter. Furthermore, for correct operation as sampler device, the one way valve 6 as mentioned in some of the embodiments described above is not present, or a two way valve 6 is present as an alternative.

In the embodiments to sampling use, the container assembly 14 may be a separate part, and disconnectable from the tubular body 1. The container assembly 14, or container 4, may be provided with a sealing (e.g. similar to the sealing 22 shown in Fig. 1) which can be applied after sampling. This allows sending the container assembly 14 or container 4 to an address (laboratory) for further diagnosis, e.g. using regular surface mail. Also, the applicator 10 may be used as sampler by removing the applicator 10 from the body cavity after sampling, and dispensing the sample in a further container which is suitable for sending to a laboratory for further diagnosis. Such containers are known as such to the skilled person.

A further embodiment is shown in cross section in Fig. 5. In this case the tubular body 1 has a tapered shape (in the cross section shown), in which the diameter d1 at the proximal end 16 is larger than the diameter d1' at the distal end 15. This embodiment of the applicator 10 has proven beneficial especially for sampling use, in actual tests virtually no leakage was observed. In an exemplary embodiment, the diameter d1 at the proximal end is 2.2 cm and the diameter at the distal end 15 is 1.4cm. Although the tapering is quite moderate (diameter from 2.2 cm to 1.4 cm), this has proven sufficient to improve sealing of the applicator 10 against the body cavity. The diameter d2 of the lumen 7 in this embodiment is only 1.3mm, which is only 6% of the diameter d1 (i.e. less than 1/3). This further improves to have as little as possible volume inside the tubular body 1. The tubular body 1 according to this embodiment may also be combined with the container assembly 14 as shown in the Fig. 1 embodiment, or with a Luer lock 17 and syringe type container 4 as shown in the Fig. 4 embodiment.

The above described alternatives of the applicator 10 are exemplary embodiments only. Further modifications of and alternatives for the various elements of the applicator 10 may be envisaged by the skilled person and are considered to be within the scope of the present invention, which is defined in the accompanying claims.

## Claims

1. Applicator device for delivery of a preparation substance to a body cavity, comprising a tubular body (1) provided with openings (8) in a distal end (15) of the tubular body (1), and a connecting opening (12) for connecting a container assembly (14) in communication with the tubular body (1),
in which the connecting opening (12) is positioned at a proximal end (16) of the tubular body (1) remote from the distal end (15), and the tubular body (1) is provided with a lumen (7) connecting the openings (8) to the connecting opening (12), a diameter (d2) of the lumen (7) being equal to or smaller than one third of a diameter (d1) of the tubular body (1) at the proximal end (16).

2. Applicator device according to claim 1, in which the diameter of the lumen (7) is less than or equal to 3mm, e.g. less than or equal to 2.2mm.

3. Applicator device according to claim 1 or 2, further comprising a plurality of channels (9) at an angle with a longitudinal axis of the tubular body (1), which channels (9) flow from the lumen (7) out in the openings (8).

4. Applicator device according to claim 3, in which a plurality of groups of channels (9) is provided, in which each group of channels (9) is at a different angle with a longitudinal axis of the tubular body (1).

5. Applicator device according to claim 3 or 4, in which a diameter of the channels (9) is less than or equal to 1mm.

6. Applicator device according to any one of claims 1-5, in which the container assembly (14) comprises a bellows type of container (4).

7. Applicator device according to any one of claims 1-6, in which the tubular body (1) and container assembly (14) further comprise a disconnectable attachment assembly (3, 20, 21).

8. Applicator device according to any one of claims 1-6, in which the tubular body (1) and container assembly (14) are fixedly attached to each other.

9. Applicator device according to any one of claims 1-6, in which the connecting opening (12) is provided with a Luer lock (17).

10. Applicator device according to any one of claims 1-9, in which the tubular body (1) further comprises a penetrating needle (2) in the connecting opening (12).

11. Applicator device according to any one of claims 1-10, in which the lumen (7) further comprises a valve (6).

12. Applicator device according to any one of claims 1-11, in which the tubular body (1) has a tapered shape, in which the diameter (d1) at the proximal end is larger than a diameter (d1') at the distal end (15).

13. Use of an applicator device according to one of the claims 1-12 for taking a sample from a body cavity.
